# EUROPEAN PATENT SPECIFICATION

(11) **EP 2 642 940 B1**
(45) Date of publication and mention of the grant of the patent: **06.01.2016**
(21) Application number: 11778781.2
(22) Date of filing: 28.10.2011
(51) Int. Cl.: A61B 19/00, F16B 2/10, F16M 13/02, A61G 13/10, F16B 2/12

(54) **BEDRAIL CLAMP**
BETTSCHIENENKLEMME
ATTACHE POUR CÔTÉ DE LIT

(30) Priority: 23.11.2010 US 416649 P; 06.07.2011 US 201113176963
(43) Date of publication of application: 02.10.2013
(73) Proprietor: Acist Medical Systems, Inc., Eden Prairie, MN 55344 (US)
(72) Inventor: RUSSELL, John, Cologne MN 55322 (US)
(74) Representative: Belloni, Giancarlo
(86) International application number: PCT/US2011/058219
(87) International publication number: WO 2012/096708

(56) References cited:
- WO-A2-2010/054836
- US-A1- 2006 290 076
- US-A1- 2010 108 841

## Description

### TECHNICAL FIELD

The present application relates generally to attaching medical accessories to a bedrail of a surgical/medical bed.

### BACKGROUND

Surgical (or related medical) procedures typically require several instruments, monitors, and other accessories. Examples of such accessories include surgical tray tables, holders for various sensors and other equipment, a contrast media injector, and so on. For many such accessories, where they are positioned in the operating room can have a significant impact on how effectively they function. Additionally, many accessories must be repositioned multiple times during the course of a surgical/medical procedure.

In many instances, it is desirable (if not required) to attach a medical accessory to a bedrail of the surgical/medical bed. For example, some sensors must be kept at a specific height relative to part of a patient's anatomy (e.g., at the same level as the midline of the patient's heart). In such instances, attaching the accessory to the bedrail of the surgical/medical bed ensures that the position of the accessory relative to that of the relevant part of the patient's anatomy remains unchanged even as the surgical/medical bed is raised and lowered during the procedure.

Bedrail clamps for attaching accessories to the bedrail have been developed, but most of them have been configured to engage the end of the bedrail and then be slid into the desired position. While such bedrail clamps may be useful in some situations, in many situations, they present significant drawbacks. A common problem with such bedrail clamps arises when multiple accessories must be attached to the bedrail in a surgical/medical procedure. For example, when two such bedrail clamps are already positioned on a bedrail, and a third bedrail clamp must be positioned between the first two,

moving the third bedrail clamp into position can be quite challenging. One of the first two bedrail clamps must first be removed by being slid to the end of the bedrail and removed. Then the third bedrail clamp must be slid from the end of the bedrail back to the desired position, and the previously attached bedrail clamp must be repositioned to its original location. This problem is only enhanced when more accessories must be attached to the same bedrail.

Some bedrail clamps have been developed in an attempt to avoid the difficulties associated with the previously mentioned bedrail clamps. Examples of such bedrail clamps are discussed in U.S. Patent No. 4,901,964 and U.S. Patent No. 7,520,007. Such bedrail clamps, however, do not provide the stability required to sufficiently support many surgical/medical accessories. Additionally, such bedrail clamps can be quite difficult to operate, resulting in even less stable attachments as well as damage to the bedrails.

Another bedrail clamp is disclosed in US 2010/108841, which describes a bedrail clamp for attaching an accessory to a bedrail of a surgical/medical bed at a desired location without having to slide the bedrail clamp from an end of the bedrail until the accessory is in the desired location, the bed rail clamp comprising:
(a) a clamp housing that includes a throat area having a first bed rail surface and being configured to receive the bedrail and a first housing guide element;
(b) an actuating member coupled to the clamp housing and configured to move relative to the clamp housing between a clamp-open position and a clamp-closed position, wherein, when the bedrail is in the clamp housing's throat area, the actuating member is nearer to the bedrail in the clamp-open position than in the clamp-closed position;
(c) a first jaw member coupled to the actuating member and housed by the clamp housing, the first jaw member including first and second bedrail portions and a first jaw member guide element configured to interface physically with the clamp housing's first housing guide element as the actuating member moves in a closing direction from the clamp-open position to the clamp-closed position to reposition the first jaw member from a first-jaw-open position in which the first jaw member does not obstruct the bedrail from entering or exiting the clamp housing's throat area to a first-jaw-closed position in which, when the bedrail is in the clamp housing's throat area, the clamp housing's first bedrail surface engages a generally vertical outer surface of the bedrail, the first jaw member's first bedrail portion is positioned proximate to a generally horizontal upper or lower surface of the bedrail, and the first jaw member's second bedrail portion engages a generally vertical inner surface of the bedrail.

### BRIEF SUMMARY

The present invention is defined in the appended claims.

Embodiments of the present invention provide a bedrail clamp with one or more jaw members positionable by an actuating member, the actuating member being configured to move away from the bedrail to move (e.g., pivot) the jaw member(s) toward the bedrail and the pull the jaw member(s) rearwardly into contact with the bedrail to secure the bedrail against the clamp housing. In many embodiments, moving the jaw member(s) toward the bedrail and then pulling the jaw member(s) into contact with the bedrail allows the bedrail clamp to accommodate bedrails of different widths. The jaw member(s) can be pulled rearwardly a relatively short distance to engage a relatively wide bedrail, and the jaw members can be pulled a relatively longer distance to engage a relatively narrow bedrail. In preferred embodiments, the bedrail clamp can accommodate bedrails with widths ranging from 6,35 mm to 12,7 mm (from 1/4 inch to 1/2 inch).

In preferred embodiments, the bedrail clamp presses against the inner and outer surfaces of the bedrail to accomplish clamping. Such embodiments distribute the clamping load across relatively large sections of the inner and outer bedrail surfaces, thereby increasing stability and avoiding more acute forces that would cause damage to the bedrail. Parts of the bedrail that are often particularly susceptible to damage caused by such acute forces are the edges of the bedrail. Applying compressive forces to the edges of the bedrail can cause them to become rounded, which can make it more difficult for conventional clamps to slide along them (along with causing other problems).

### BRIEF DESCRIPTION OF THE FIGURES

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.
FIG. 1 is a surgical/medical bed with various accessories attached to the bedrail.
FIG. 2A is a side schematic view of a single-jaw bedrail clamp in accordance with an embodiment of the invention, with half of the clamp housing removed and the bedrail clamp's jaw member in a jaw-open position.
FIG. 2B is a side schematic view of the single-jaw bedrail clamp of FIG. 2A, with the bedrail clamp's jaw member in a jaw-partially-closed position.
FIG. 2C is a side schematic view of the single-jaw bedrail clamp of FIG. 2A, with the bedrail clamp's jaw member in a jaw-closed position.

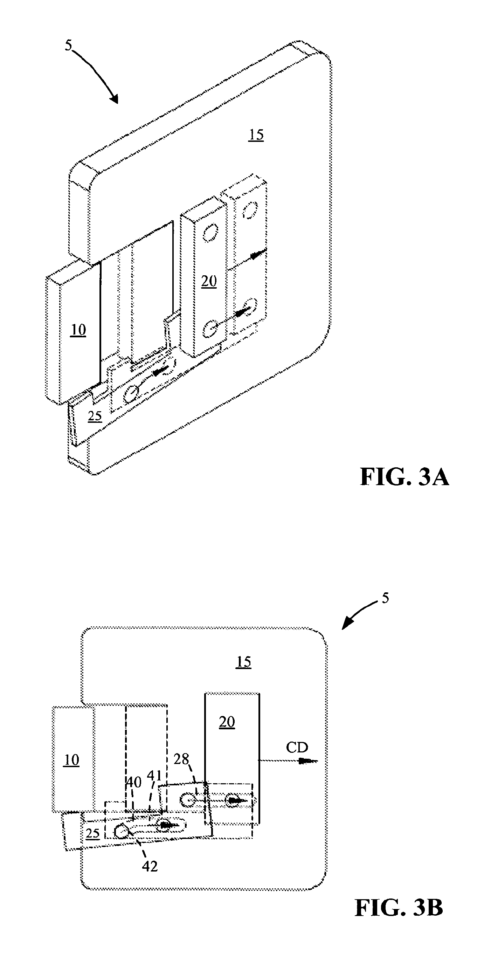

FIG. 3A is a perspective view of a single-jaw bedrail clamp in accordance with an embodiment of the invention, with half of the clamp housing removed.
FIG. 3B is a side view of the single-jaw bedrail clamp of FIG. 3A.
FIG. 4 is an exploded view of a double-jaw bedrail clamp in accordance with an embodiment of the invention.
FIG. 5A is a perspective view of a double-jaw bedrail clamp in accordance with an embodiment of the present invention, with half of the clamp housing removed.
FIG. 5B is a side view of the double-jaw bedrail clamp of FIG. 5A.
FIG. 6A is a side schematic view of a double-jaw bedrail clamp in accordance with an embodiment of the invention, with half of the clamp housing removed and the bedrail clamp's jaw members in jaw-open positions.
FIG. 6B is a side schematic view of the double-jaw bedrail clamp of FIG. 6A, with the bedrail clamp's jaw members in jaw-partially-closed positions.
FIG. 6C is a side schematic view of the double-jaw bedrail clamp of FIG. 6A, with the bedrail clamp's jaw members in jaw-closed positions.
FIG. 7 is a perspective view of a bedrail clamp in accordance with an embodiment of the invention.
FIG. 8A is a side schematic view of part of a single-jaw bedrail clamp in accordance with an embodiment of the invention, with the bedrail clamp's jaw member in jaw-open position.
FIG. 8B is a side schematic view of the part of the single-jaw bedrail clamp of FIG. 8A, with the bedrail clamp's jaw member in a jaw-partially-closed position.
FIG. 8C is a side schematic view of the part of the single-jaw bedrail clamp of FIG. 8A, with the bedrail clamp's jaw member in a jaw-closed position.

### DESCRIPTION

The following detailed description is exemplary in nature and is not intended to limit the scope, applicability, or configuration of the invention in any way. Rather, the following description provides practical illustrations for implementing exemplary embodiments of the present invention. Examples of constructions, materials, dimensions, and manufacturing processes are provided for selected elements, and all other elements employ that which is known to those of skill in the field of the invention. Those skilled in the art will recognize that many of the examples provided have suitable alternatives that can be utilized.

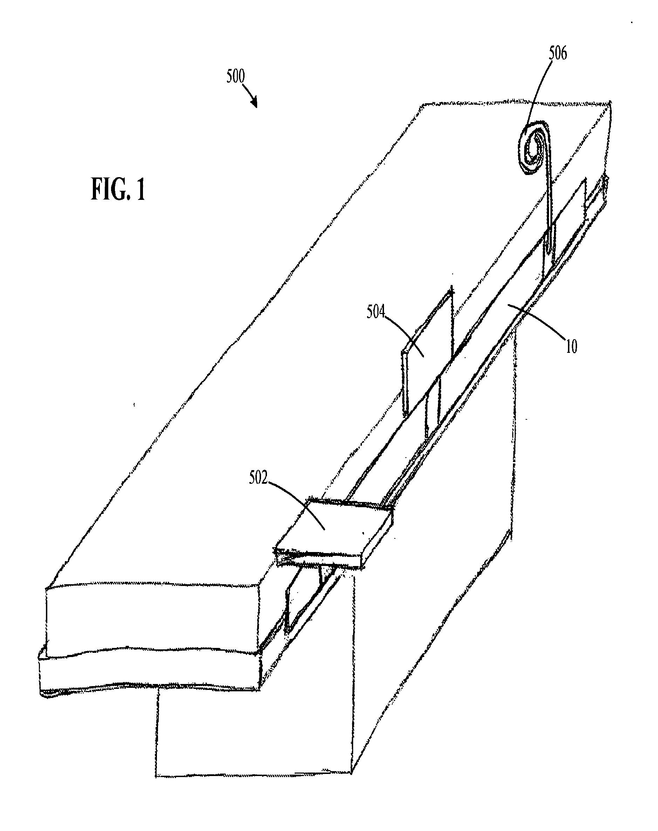

FIG. 1 shows an illustrative operating room with a surgical/medical bed 500 having a bedrail 10 to which various accessories (tray table 502, monitor 504, and holder 506) are attached. As can be seen, if only conventional bedrail clamps were available, and if an additional accessory needed to be positioned between monitor 504 and holder 506, holder 506 would first have to be slid to the end 508 of the bedrail 10 and removed. Only then could the additional accessory be positioned between monitor 504 and holder 506. And after that, holder 506 would have to be engaged to the end 508 of the bedrail 10 and slid back into position. A similar situation would arise if monitor 504 needed to be repositioned to the other side of tray table 502. In that situation, both tray table 502 and monitor 504 must be slid to the end 510 of bedrail 10 and removed and then their positions reversed.

Embodiments of the present invention allow positioning and repositioning of accessories at any open position along the bedrail 10 without changing the position of any accessories that are already attached to the bedrail 10. In the examples discussed above, if an additional accessory needed to be positioned between monitor 504 and holder 506, it could simply be attached in that position without even addressing the position of monitor 504 and holder 506. Likewise, if monitor 504 needed to be repositioned to the other side of tray table 502, monitor 504 could simply be removed from its current position and attached on the other side of tray table 502 without even addressing the position of tray table 502.

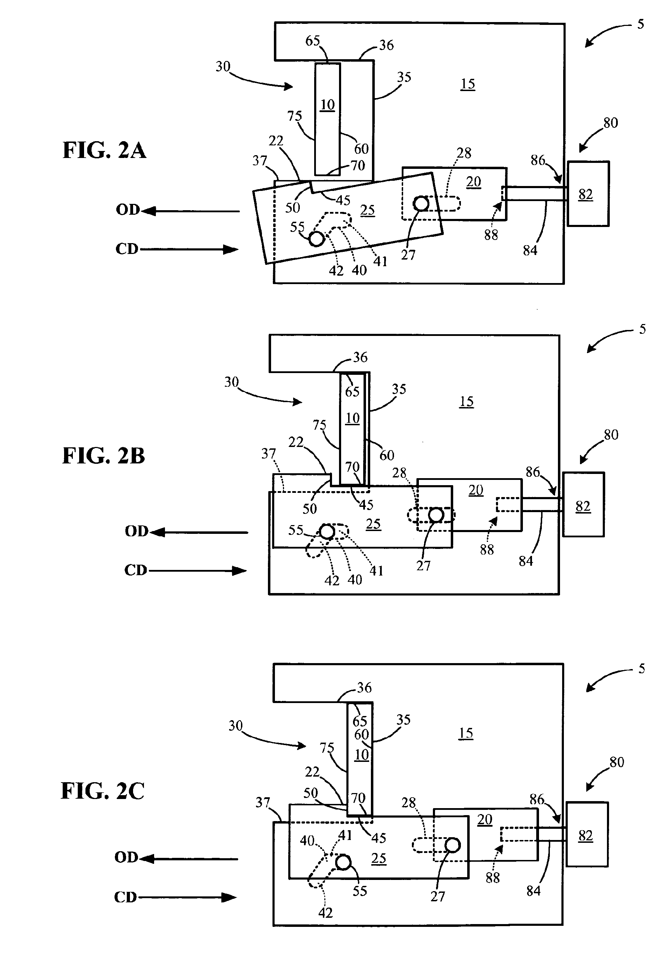

FIGS. 2A-2C show a bedrail clamp 5 according to embodiments of the present invention. The bedrail clamp 5 can attach an accessory to a bedrail 10 of a surgical/medical bed at a desired location without having to slide the bedrail clamp 5 from an end of the bedrail 10 until the accessory is in the desired location. As noted, the bedrail clamp 5 can be positioned at any location along the bedrail 10 that is not otherwise occupied, and the positioning can occur without having to alter the location of any other accessories or associated bedrail clamps that may be attached to the bedrail 10. The bedrail clamp 5 of FIGS. 2A-2C includes a clamp housing 15, an actuating member 20 coupled to the clamp housing 15, and a jaw member 25 pivotably coupled to the actuating member 20 and housed by the clamp housing 15.

As its name suggests, the clamp housing 15 can house other components of the bedrail clamp 5. In many embodiments, the clamp housing 15 is generally C-shaped and includes a throat area 30. When the bedrail clamp 5 is to be attached to the bedrail 10, the throat area 30 can receive the bedrail 10, and then the bedrail clamp 5 can be closed (as is discussed in greater detail below). The throat area 30 of the clamp housing 15 shown in FIGS. 2A-2C includes three bedrail surfaces 35, 36, 37. The clamp housing 15 can include a housing guide element, which is discussed in greater detail below.

The actuating member 20 of the bedrail clamp 5 can coordinate the opening and closing of the bedrail clamp 5. The actuating member 20 can be configured to move relative to the clamp housing 15 between a clamp-open position (FIG. 2A) and a clamp-closed position (FIG. 2C). When the bedrail 10 is in the clamp housing's throat area 30, the actuating member 20 can be nearer to the bedrail 10 in the clamp-open position than in the clamp-closed position. Accordingly, to go from the clamp-open position to the clamp- closed position, the actuating member 20 must move in a closing direction, which is indicated as CD in FIGS. 2A-2C. Also, to go from the clamp-closed position to the clamp-open position, the actuating member 20 must move in an opening direction, which is indicated as OD in FIGS. 2A-2C. In preferred embodiments, the closing direction CD, the opening direction OD, or both are generally horizontal.

The jaw member 25 of the bedrail clamp 5 can move between a jaw-open position and a jaw-closed position based on the movement of the actuating member 20. The jaw member 25 includes bedrail portions 45, 50 that are configured to interact with the bedrail 10 when in use. As shown, bedrail portion 45 has a first surface, and bedrail portion 50 has a second surface that is generally perpendicular to the first surface. Bedrail portion 45 can be configured to be positioned proximate to the bedrail 10 (i.e., contacting the bedrail 10 or positioned near the bedrail 10 without contacting it) when the jaw member 25 is in the jaw-closed position. Bedrail portion 50 can be configured to engage the bedrail 10 when the jaw member 25 is in the jaw-closed position. The jaw member 25 can include a guide element, which is discussed in greater detail below.

FIG. 2A shows the jaw member 25 in the jaw-open position. As can be seen, the jaw member 25 does not obstruct the bedrail 10 from entering or exiting the clamp housing's throat area 30. When the bedrail clamp 5 is oriented as shown in FIGS. 2A-2C, the entrance tip 22 of the jaw member 25 can be positioned at the same level as or below the clamp housing's bedrail surface 37 where the bedrail 10 would enter the throat area 30. In many embodiments, in the jaw-open position, entrance tip 22 of the jaw member 25 can be spaced vertically far enough from the clamp housing's bedrail surface 36 to permit the bedrail 10 to enter the throat area 30. Two common bedrail heights are 2,54 centimeters (one inch) and twenty-five centimeters. In some preferred embodiments, the height of the clamp housing's throat area 30 is slightly larger than those common bedrail heights. In many such embodiments, the vertical distance from bedrail surface 36 to bedrail surface 37 is slightly larger than those common bedrail heights. In some preferred embodiments, the vertical distance from the clamp housing's bedrail surface 36 to the entrance tip 22 of the jaw member 25 is slightly larger than the common bedrail heights when the jaw member 25 is in the jaw-open position.

In preferred embodiments, the jaw member 25 can move to a jaw-partially-closed position, which, as shown in FIG. 2B, is between the jaw-open position and the jaw-closed position. In the jaw-partially-closed position, the jaw member 25 obstructs the bedrail 10, when positioned in the clamp housing's throat area 30, from exiting the clamp housing's throat area 30. The entrance tip 22 of the jaw member 25 can be brought to a position such that the vertical distance between the entrance tip 22 and bedrail surface 36 of the clamp housing 15 is less than the height of the bedrail 10. But, in the jaw-partially-closed position, the jaw member 25 of the bedrail clamp 5 does not fully engage the bedrail 10. In other words, either the clamp housing's bedrail surface 35 does not engage the outer surface 60 of the bedrail 10 or bedrail portion 50 of the jaw member 25 does not engage the inner surface 75 of the bedrail 10 (or both). In some instances, the bedrail clamp 5 can be rotated when the jaw member 25 is in the jaw-partially-closed position such that the bedrail 10 can still be removed from the throat area 30. The amount of space between bedrail surface 35 and outer surface 60 plus the amount of space between bedrail portion 50 and inner surface 75 is how far the jaw member 25 must be translated in the closing direction to move the jaw member to the jaw-closed position.

FIG. 2C shows the jaw member 25 in the jaw-closed position. As can be seen, the jaw member 25 and the clamp housing 15 can engage the bedrail 10 when the bedrail 10 is positioned in the clamp housing's throat area 30. The clamp housing's bedrail surface 35 can engage a generally vertical outer surface 60 of the bedrail 10. Bedrail portion 45 of the jaw member 25 can be positioned proximate to a generally horizontal upper surface 65 or a generally horizontal lower surface 70 of the bedrail 10. In other words, the bedrail clamp 5 can be used as oriented in FIGS. 2A-2C, with the jaw member 25 positioned proximate to the lower surface 70 of the bedrail 10, or the bedrail clamp 5 can be turned upside down such that the jaw member 25 is positioned proximate to the upper surface 65 of the bedrail 10. Bedrail surface 36 of the clamp housing 15 can be positioned proximate to whichever of the upper or lower surfaces 65, 70 of the bedrail 10 is not positioned proximate to bedrail portion 45. In many situations, the orientation of FIGS. 2A-2C, with bedrail surface 36 positioned proximate to the upper surface 65 and bedrail portion 45 positioned proximate to the lower surface 70, is the preferred orientation. As alluded to above, bedrail portion 50 of the jaw member 25 can engage a generally vertical inner surface 75 of the bedrail 10 in the jaw-closed position.

In many embodiments, closing the bedrail clamp 5 involves applying force to at least the inner and outer surfaces 75, 60 of the bedrail 10. As alluded to above, in preferred embodiments, bedrail portions 45, 50 of the jaw member 25 each comprise generally flat surfaces that are perpendicular to one another. In this way, bedrail portion 50 can be substantially flush with the inner surface 75 of the bedrail 10, and bedrail surface 36 of the clamp housing 15 can be substantially flush with the upper surface 65 (or lower surface 70) of the bedrail 10. In this way, the clamping load is distributed across relatively large sections of the inner and outer bedrail surfaces 75, 60, thereby increasing stability and avoiding more acute forces that would cause damage to the bedrail. Many configurations are possible to satisfactorily secure the bedrail clamp 5 to the bedrail 10.

The bedrail clamp 5 of FIGS. 2A-2C further includes a handle assembly 80, which can permit a user to operate the bedrail clamp 5. As shown, the handle assembly 80 includes a handle 82 and a handle shaft 84 housed by the clamp housing 15. The handle shaft 84 can have a proximal end 86 coupled to the handle 82. The handle shaft 84 can have a distal end 88 coupled to the actuating member 20.

The handle assembly 80 can take a variety of forms. In some embodiments, the handle assembly 80 is attached to the clamp housing 15 and achieves relative movement between the actuating member 20 and the clamp housing 15 by causing movement of the actuating member 20 relative to the handle assembly 80. An example of such an embodiment is shown in FIGS. 2A-2C, with the handle 82 comprising a knob, and the distal end 88 of the handle shaft 84 being threadedly coupled to the actuating member 20. In such embodiments, turning the knob can cause the actuating member 20 to move between the clamp-open position and the clamp-closed position.

In some embodiments, the handle assembly can be attached to the actuating member and can achieve relative movement between the actuating member and the clamp housing by causing movement of the clamp housing relative to the handle assembly. For example, the handle shaft and the clamp housing can include complementary springloaded ratchet components, which can be configured to permit the handle and the handle shaft to move the actuating member between the clamp-open position and the clamp-closed position. In some such embodiments, the handle and the handle shaft are configured to move the actuating member in a ratcheting manner from the clamp-open position to the clamp-closed position. In such embodiments, the handle and the handle shaft can be configured to move the actuating member via a spring force from the clamp-closed position to the clamp-open position upon release of the complementary springloaded ratchet components. In another example, the handle can comprise a camming latch that is configured to cam against the clamp housing to move between a camming-latch-open position and a camming-latch-closed position. In some such embodiments, the camming latch is configured to cause the actuating member to move from the clamp-open position to the clamp-closed position as the camming latch moves from the camming-latch-open position to the camming-latch-closed position.

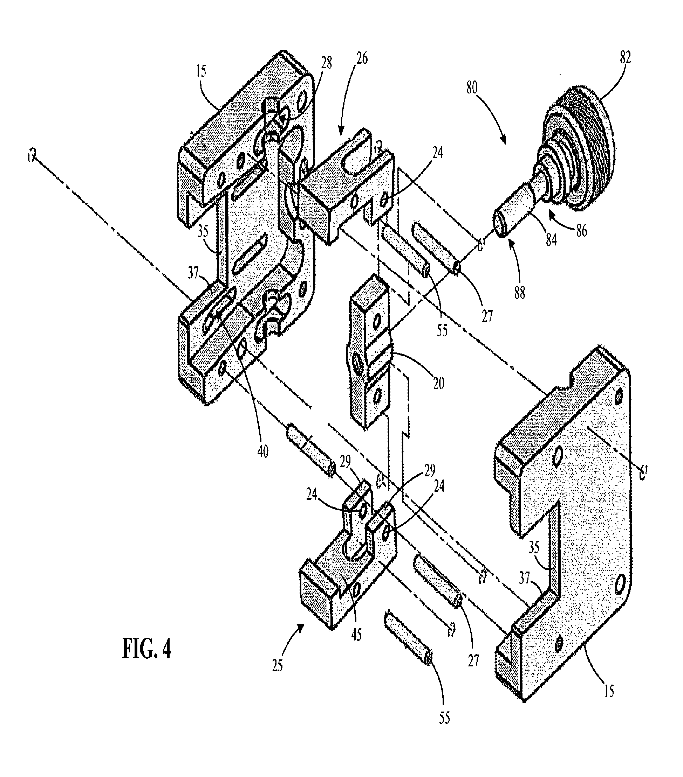

The pivotable coupling between the jaw member 25 and the actuating member 20 can take a variety of forms. The pivotable coupling is discussed with reference to FIG. 4, which illustrates configurations that can be implemented in single-jaw or double-jaw bedrail clamps. The jaw member 25 can include a pivot hole 24 that extends generally parallel to bedrail surface 35 of the clamp housing 15. In some such embodiments, the actuating member 20 can include a pivot pin 27 that extends into the jaw member's pivot hole 24 to pivotably couple the jaw member 25 to the actuating member 20. The pivot pin 27 can be integral with the jaw member 25, integral with the actuating member 20, or its own separate component (in which case both the jaw member 25 and the actuating member would have pivot holes). In some preferred embodiments, the jaw member 25 can include two attachment members 29 that are configured to straddle the actuating member 20, with a separate hinge pin providing the coupling that allows the jaw member 25 to pivot relative to the actuating member. The jaw member's attachment members 29 straddling the actuating member 20 can minimize forces that would tend to rotate the actuating member 20 about a horizontal axis that extends between the jaw-open position and the jaw-closed position. The exploded view of FIG. 4 shows a preferred arrangement of the pivot pin 27, the pivot hole 24, the attachment members 29 in connection with the actuating member 20 and the jaw member 25.

Referring again to FIGS. 2A-2C, both the jaw member 25 and the actuating member 20 can translate relative to the clamp housing 15. In some preferred embodiments, with a pivot pin 27 enabling the jaw member 25 to pivot relative to the actuating member 20, the clamp housing 15 can include a recessed track 28 that extends generally parallel to the closing direction CD. In some such embodiments, the pivot pin 27 can extend beyond the jaw member 25 into the clamp housing's recessed track 28. In some such embodiments, the pivot pin 27 can be configured to slide within the clamp housing's recessed track 28 to permit the actuating member 20 and the jaw member 25 to translate relative to the clamp housing 15. In some embodiments, the structure of the clamp housing 15 can be configured to guide the jaw member 25 and the actuating member 20 in translating between open and closed positions.

In many embodiments, movement of the jaw member 25 relative to the clamp housing 15 can be directed by the jaw member's guide element interfacing physically with the clamp housing's guide element. As shown in FIGS. 2A-2C, the clamp housing's guide element is a recessed track 40, and the jaw member's guide element is a guide pin 55. The exploded view of FIG. 4 shows a preferred arrangement of the recessed track 40 and the guide pin 55 (though in the context of a double-jaw bedrail clamp). Referring again to FIGS. 2A-2C, movement of the jaw member 25 relative to the clamp housing 15 is guided by the guide pin 55 sliding within the recessed track 40. In preferred embodiments, the recessed track 40 can include a first segment 41 that is generally parallel to the closing direction CD and a second segment 42 that forms a generally obtuse angle with the first segment 41. In some embodiments, the second segment 42 of the recessed track 40 can be curved. In such embodiments, the first segment 41 would typically remain straight to guide translation of the jaw member 25 relative to the clamp housing 15. As noted above, movement is typically guided by the guide elements from the jaw-open position to the jaw-partially-closed position to the jaw-closed position (and in reverse as well).

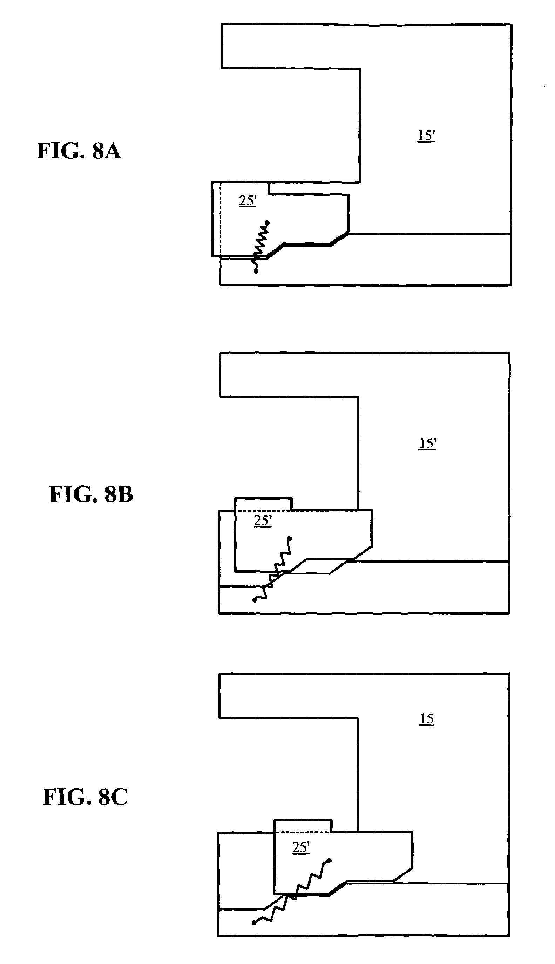

The clamp housing's guide element and the jaw member's guide element can take a variety of forms. For example, the jaw member 25 can include a recessed track, and the clamp housing 15 can include a guide pin. In another example, both the jaw member 25 and the clamp housing 15 can include a recessed track, and one or more ball bearings can be positioned within both recessed tracks to guide movement. The structure of the clamp housing 15 and the structure of the jaw member 25 can include complementary features (e.g., curves, angled segments, etc.) to guide movement as the actuating member 20 moves. FIGS. 8A-8C provide an example of such a configuration. In FIGS. 8A-8C, the clamp housing 15' can include a guiding rail defined in its interior, and the jaw member 25' structure itself can ride along that rail between a jaw-open position and a jaw-closed position. As can be seen, a spring 23 can assist in moving from the jaw-closed position to the jaw-open position by. The configuration of FIGS.8A-8C and other similar configurations (e.g., those that involve pivoting the jaw member(s)) can be incorporated into a double-jaw clamp as well.

In use, both the actuating member 20 and the jaw member 25 can be moved between open and closed positions. As the actuating member 20 moves in a closing direction CD from the clamp-open position to the clamp-closed position, the physical interfacing of the jaw member's guide element and the clamp housing's guide element can reposition the jaw member 25 from the jaw-open position to the jaw-closed position. Such physical interfacing can both translate the jaw member 25 in the closing direction CD and pivot the jaw member 25 to reposition the jaw member 25 from the jaw-open position to the jaw-closed position. In many embodiments, the first movement of the jaw member 25 involves both translation and rotation, and the second movement of the jaw member 25 involves only translation. As shown in FIGS. 2A-2C, the guide pin 55 slides within the recessed track 40 between the jaw-open position and the jaw-closed position.

In some such embodiments, movement of the actuating member 20 in the closing direction CD repositions the jaw member 25 first from the jaw-open position to the jaw-partially-closed position and then from the jaw-partially-closed position to the jaw-closed position. In preferred embodiments, repositioning the jaw member 25 from the jaw-open position to the jaw-partially-closed position involves both translating the jaw member 25 in the closing direction CD and pivoting the jaw member 25. In some such embodiments, pivoting the jaw member 25 can involve overcoming a spring force that would resist such rotation. In preferred embodiments, repositioning the jaw member 25 from the jaw-partially-closed position to the jaw-closed position involves only translating the jaw member 25 in the closing direction CD. As noted above, FIG. 2A shows the jaw member 25 in the jaw-open position, FIG. 2B shows the jaw member 25 in the jaw-partially-closed position, and FIG. 2C shows the jaw member 25 in the jaw-closed position.

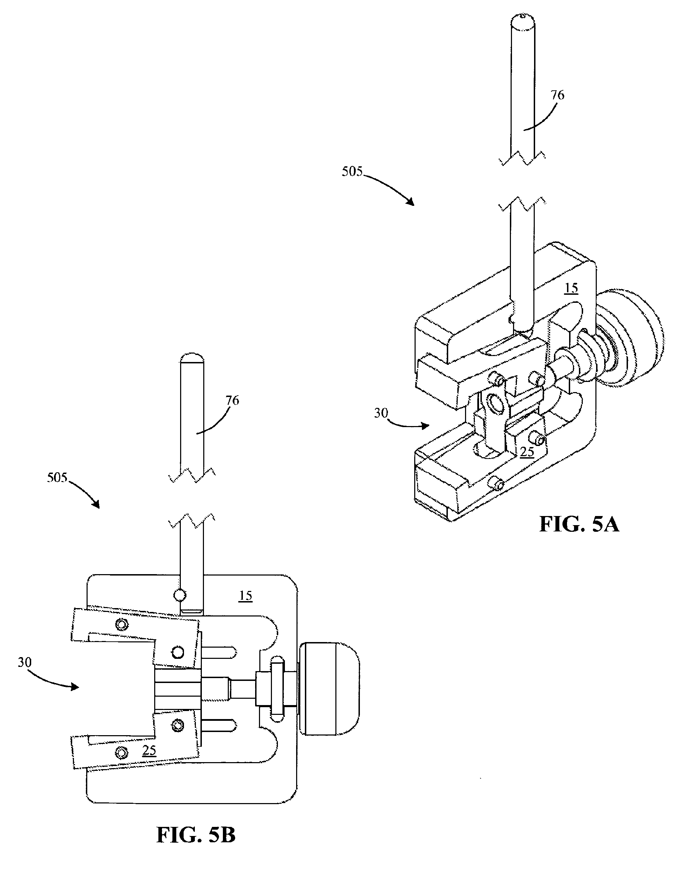

FIGS. 5A-5B show a the bedrail clamp 505, with an accessory attachment support 76 that is configured to support an accessory, such as those discussed elsewhere herein. As shown, the accessory attachment support 76 includes a proximal end housed by the clamp housing 15. The accessory attachment support 76 also includes a distal end that extends away from the clamp housing 15 above the bedrail 10 when the bedrail 10 is in the clamp housing's throat area 30 and the jaw member 25 is in the first-jaw-closed position. In some embodiments, the accessory can be pre-attached to the bedrail clamp. In some embodiments, the accessory must be attached to the bedrail clamp after the bedrail clamp is attached to the bedrail.

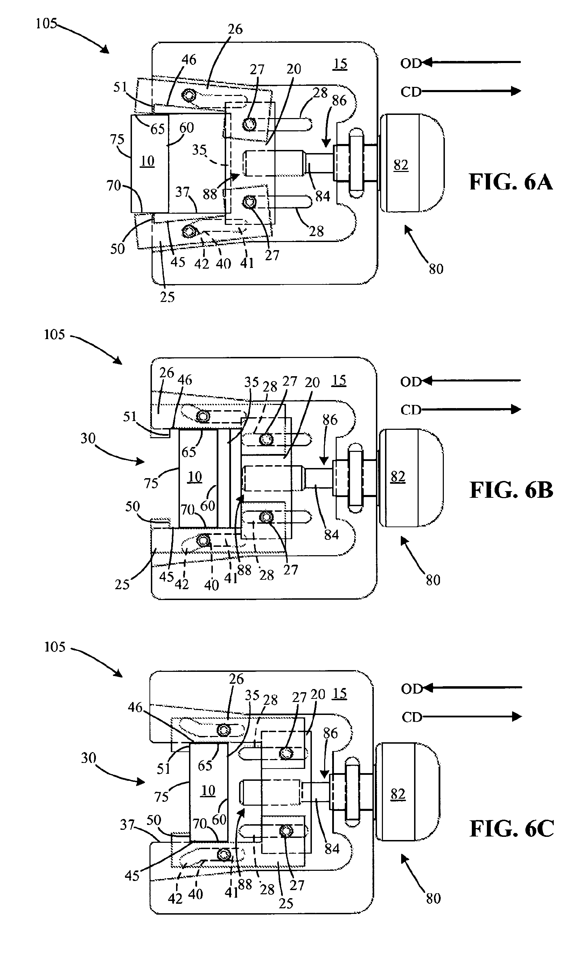

FIGS. 6A-6C show a bedrail clamp 105 similar to that of FIGS. 2A-2C, with the exception that bedrail clamp 105 includes two jaw members 25, 26. Like jaw member 25, jaw member 26 of FIGS. 6A-6C is pivotably coupled to the actuating member 20 and housed by the clamp housing 15. The pivotable coupling of jaw member 26 and the actuating member 20 can have similar attributes as discussed elsewhere herein in connection with the pivotable coupling of jaw member 25 and the actuating member 20. Jaw member 26 can move between a jaw-open position (FIG. 6A), a jaw-partially-closed position (FIG. 6B), and a jaw-closed position (FIG. 6C) based on the movement of the actuating member 20. As shown, jaw member 26 includes bedrail portions 46, 51 that are configured to interact with the bedrail 10 when in use. Jaw member 26 can include a guide element, which can interface physically with a corresponding guide element in the clamp housing 15 when in use. The guide element of jaw member 26 and the guide element of the clamp housing 15 can have similar attributes as guide elements discussed elsewhere herein. In most embodiments, the guide element combination of jaw member 25 and the clamp housing 15 is the same as the guide element combination of jaw member 26 and the clamp housing, though the two guide element combinations can be different.

The jaw-open position and jaw-closed position of jaw member 26 are similar to those of jaw member 25, as discussed above in connection with FIGS. 2A-2C. Referring again to FIGS. 6A-6C, in the jaw-open position, jaw member 26 does not obstruct the bedrail 10 from entering or exiting the clamp housing's throat area 30. In the jaw-closed position, the clamp housing's bedrail surface 35 can engage the outer surface 60 of the bedrail 10. Additionally, bedrail portion 46 of jaw member 26 can be positioned proximate to whichever of the upper surface 65 or the lower surface 70 of the bedrail 10 that is not positioned proximate to bedrail portion 45 of jaw member 25 when in a jaw-closed position. In addition, bedrail portion 51 can also engage the inner surface 75 of the bedrail 10.

In use, the bedrail clamp 105 of FIGS. 6A-6C operates similarly to that of FIGS. 2A-2C. Referring again to FIGS. 6A-6C, as the actuating member 20 moves in the closing direction CD from the clamp-open position to the clamp-closed position, both jaw member 25 and jaw member 26 are repositioned from jaw-open positions to jaw-closed positions. The guide element of jaw member 25 can physically interface with the clamp housing's first guide element to reposition jaw member 25, and the guide element of jaw member 26 can physically interface with the clamp housing's second guide element to reposition jaw member 26. In both cases, such repositioning can be via both translating the jaw members 25, 26 in the closing direction CD and pivoting the jaw members 25, 26.

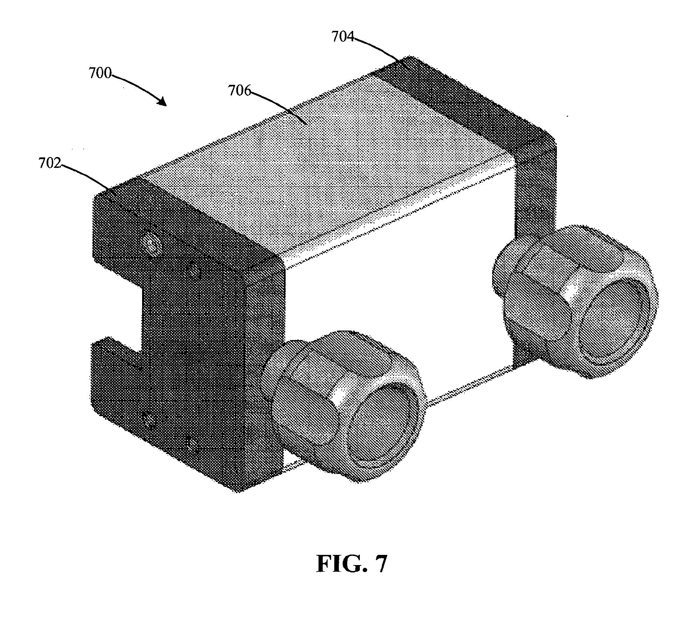

FIG. 7 shows a bedrail clamp assembly 700 according to embodiments of the present invention. As can be seen, the bedrail clamp assembly 700 includes two clamps 702, 704 separated by a C-block 706. Clamps 702, 704 can be structured and can operate similarly to other clamps discussed herein (e.g., bedrail clamp 5 of FIGS. 2A-2C, bedrail clamp 105 of FIGS. 6A-6C, etc.). Referring again to FIG. 7, bedrail clamp assembly 700 can be used to clamp larger accessories to a bedrail in a medical setting. An example of such a larger accessory is a contrast media injector. Given its larger contact area and its multiple clamps 702, 704, bedrail clamp 700 can provide significantly increased stability in supporting larger accessories. The distance between the clamps 702, 704, and thus the size of the C-block 706 can be determined based on the particular application. In some embodiments, spacer members other than a C-block (e.g., rods, top and bottom support members, etc.) can be used.

Aspects of the present invention involve methods of attaching an accessory to a bedrail of a surgical/medical bed at a desired location without having to slide the accessory from an end of the bedrail to the desired location. A bedrail clamp, such as those discussed herein, can be provided in a surgical/medical environment. In some instances, the accessory is attached to the bedrail clamp before the bedrail clamp is attached to the bedrail. In some instances, the accessory must be attached to the bedrail clamp after the bedrail clamp is attached to the bedrail. In either case, an operator can ensure that the actuating member of the bedrail clamp is in the clamp-open position and that any jaw members are in jaw-open positions. Clamp-open positions of the actuating member and jaw-open positions of the jaw member(s) are discussed in greater detail elsewhere herein. The operator can introduce the bedrail into the throat area of the bedrail clamp's clamp housing without obstruction from the jaw member(s). The operator can do so in accordance with discussion elsewhere herein.

With the bedrail in the clamp housing's throat area, the operator can close the bedrail clamp. The operator can move the actuating member in a closing direction from a clamp-open position to a clamp-closed position. In so doing, the jaw member(s) can be repositioned from the jaw-open position to a jaw-closed position. Such repositioning of the jaw member(s) can involve both translating the jaw member(s) in the closing direction and pivoting the jaw member(s). Specific ways of accomplishing such positioning (e.g., via guide elements) are discussed elsewhere herein.

The jaw-closed position can involve a bedrail surface of the clamp housing engaging a generally vertical outer surface of the bedrail. Attributes of some jaw-closed positions are discussed elsewhere herein. In many cases, when a single-jaw bedrail clamp is used, the jaw-closed position can involve a bedrail portion of the lone jaw member being positioned proximate to either a generally horizontal upper or lower surface of the bedrail and a second bedrail surface of the clamp housing being positioned proximate to whichever of the upper or lower surfaces of the bedrail is not positioned proximate to jaw member's bedrail portion. In preferred methods of using single-jaw bedrail clamps, the clamp housing's second bedrail surface is positioned proximate to the bedrail's upper surface, and the lone jaw member's bedrail portion is positioned proximate to the bedrail's lower surface. Further, the jaw-closed position can involve a second bedrail portion of the lone jaw member engaging a generally vertical inner surface of the bedrail.

When a double-jaw bedrail clamp is used, the second jaw member can interact with the bedrail in the jaw-closed position. As in methods of using single-jaw bedrail clamps, when a double-jaw bedrail clamp is used, the jaw-closed position can involve the clamp housing's bedrail surface engaging the outer surface of the bedrail and the first jaw member's bedrail portions being positioned proximate to the upper/lower surface of the bedrail and engaging the inner surface of the bedrail, respectively. In methods of using double-jaw bedrail clamps, the jaw-closed position can further involve a bedrail portion of the second jaw member being positioned proximate to whichever of the upper or lower surfaces of the bedrail is not positioned proximate to the first jaw member's first bedrail portion. In preferred embodiments, the operator need not determine which jaw member is supposed to be positioned proximate to the bedrail's upper surface and which jaw member is supposed to be positioned proximate to the bedrail's lower surface because the bedrail clamp is substantially symmetrical, with both jaw members being configured to be positioned proximate to either surface of the bedrail. Additionally, in such methods, the jaw-closed position can further involve a second bedrail portion of the second jaw member engaging the inner surface of the bedrail.

In many preferred methods, repositioning the jaw member(s) can involve moving the jaw member(s) through a jaw-partially-closed position, which is between the jaw-open position and the jaw-closed position. Attributes of some jaw-partially-closed positions are discussed elsewhere herein. In the jaw-partially-closed position, the jaw member(s) can obstruct the bedrail, when positioned in the clamp housing's throat area, from exiting the clamp housing's throat area. But, in the jaw-partially-closed position, the jaw member(s) do not fully engage the bedrail. In other words, either the clamp housing's bedrail surface does not engage the outer surface of the bedrail or the second bedrail portion of the jaw member(s) does not engage the inner surface of the bedrail (or both). The jaw member(s) can first be repositioned from the jaw-open position to the jaw-partially-closed position. As alluded to elsewhere herein, such repositioning can involve both translating the jaw member(s) in the closing direction and pivoting the jaw member(s). Then, the jaw member(s) can be repositioned from the jaw-partially-closed position to the jaw-closed position. Such repositioning can involve only translating the jaw member(s) in the closing direction. In this way, using a single-jaw bedrail clamp, the jaw member can first be closed to its final distance from the generally horizontal bedrail surface of the clamp housing, and then the jaw member can be pulled back to press the bedrail against the generally vertical bedrail surface of the clamp housing, thereby accommodating bedrails of varying lengths.

In the foregoing detailed description, the invention has been described with reference to specific embodiments. However, it may be appreciated that various modifications and changes can be made without departing from the scope of the invention as set forth in the appended claims. Thus, some of the features of preferred embodiments described herein are not necessarily included in preferred embodiments of the invention which are intended for alternative uses.

## Claims

1. A bedrail clamp (5) for attaching an accessory to a bedrail (10) of a surgical/medical bed at a desired location without having to slide the bedrail clamp from an end of the bedrail until the accessory is in the desired location, the bedrail clamp comprising:
(a) a clamp housing (15) that includes (i) a throat area (30) having a first bedrail surface (35) and being configured to receive the bedrail and (ii) a first housing guide element (40);
(b) an actuating member (20) coupled to the clamp housing and configured to move relative to the clamp housing between a clamp-open position and a clamp-closed position, wherein, when the bedrail is in the clamp housing's throat area, the actuating member is nearer to the bedrail in the clamp-open position than in the clamp-closed position;
(c) a first jaw member (25) coupled to the actuating member and housed by the clamp housing, the first jaw member including (i) first (45) and second (50) bedrail portions and (ii) a first jaw member guide element (55) configured to interface physically with the clamp housing's first housing guide element as the actuating member moves in a closing direction from the clamp-open position to the clamp-closed position to reposition the first jaw member from (A) a first-jaw-open position in which the first jaw member does not obstruct the bedrail from entering or exiting the clamp housing's throat area to (B) a first jaw-partially-closed position and then, via only translating the first jaw member in the closing direction, to (C) a first-jaw-closed position in which, when the bedrail is in the clamp housing's throat area, the clamp housing's first bedrail surface engages a generally vertical outer surface (60) of the bedrail, the first jaw member's first bedrail portion (45) is positioned proximate to a generally horizontal upper (65) or lower (70) surface of the bedrail, and the first jaw member's second bedrail portion (50) engages a generally vertical inner surface (75) of the bedrail.

2. The bedrail clamp of claim 1, wherein (i) the clamp housing's throat area further has a second bedrail surface (36) and (ii) when the first jaw member is in the first-jaw-closed position and when the bedrail is in the clamp housing's throat area, the clamp housing's second bedrail surface is positioned proximate to whichever of the generally horizontal upper or lower surfaces of the bedrail is not positioned proximate to the first jaw member's first bedrail portion.

3. The bedrail clamp of claim 1, wherein the first housing guide element comprises a first recessed track (40) and the first jaw member guide element comprises a first guide pin (55) configured to slide within the first recessed track as the actuating member moves between the clamp-open position and the clamp-closed position.

4. The bedrail clamp of claim 3, wherein the first recessed track comprises a first segment (41) that is generally parallel to the closing direction and a second segment (42) that forms a generally obtuse angle with the first segment.

5. The bedrail clamp of claim 1, wherein the first-jaw-partially-closed position is such that the first jaw member does obstruct the bedrail, when positioned in the clamp housing's throat area, from exiting the clamp housing's throat area, but the clamp housing's first bedrail surface does not engage the generally vertical outer surface of the bedrail and/or the first jaw member's second bedrail portion does not engage the generally vertical inner surface of the bedrail.

6. The bedrail clamp of claim 1, wherein the clamp housing further includes (iii) a second housing guide element, and wherein the bedrail clamp further comprises (d) a second jaw member (26) coupled to the actuating member and housed by the clamp housing, the second jaw member including (i) third (46) and fourth (51) bedrail portions and (ii) a second jaw member guide element configured to interface physically with the clamp housing's second housing guide element as the actuating member moves in the closing direction from the clamp-open position to the clamp-closed position to reposition the second jaw member, via both translating the second jaw member in the closing direction and moving the second jaw member, from (A) a second-jaw-open position in which the second jaw member does not obstruct the bedrail from entering or exiting the clamp housing's throat area to (B) a second-jaw-closed position in which, when the bedrail is in the clamp housing's throat area, the clamp housing's first bedrail surface engages the generally vertical outer surface of the bedrail, the second jaw member's third bedrail portion is positioned proximate to whichever of the generally horizontal upper or lower surfaces of the bedrail is not positioned proximate to the first jaw member's first bedrail portion when the first jaw member is in the first-jaw-closed position, and the second jaw member's fourth bedrail portion engages the generally vertical inner surface of the bedrail.

7. The bedrail clamp of claim 1, wherein the closing direction is generally horizontal.

8. The bedrail clamp of claim 1, wherein the first jaw member's first bedrail portion comprises a first surface and the first jaw member's second bedrail portion comprises a second surface that is generally perpendicular to the first surface.

9. The bedrail clamp of claim 1, further comprising a handle assembly (80), which includes a handle (82) and a handle shaft (84) housed by the clamp housing, the handle shaft having a proximal end (86) coupled to the handle and a distal end (88) coupled to the actuating member (20).

10. The bedrail clamp of claim 9, wherein the handle comprises a knob and the distal end of the handle shaft is threadedly coupled to the actuating member such that turning the knob causes the actuating member to move between the clamp-open position and the clamp-closed position.

11. The bedrail clamp of claim 1, wherein the first jaw member is pivotably coupled to the actuating member, and the first jaw member guide element is configured to interface physically with the first housing guide element as the actuating member moves in the closing direction from the clamp-open position to the clamp-closed position to reposition the first jaw member, via both translating the first jaw member in the closing direction and pivoting the first jaw member, from the first-jaw-open position to the first-jaw-closed position.

12. The bedrail clamp of claim 11, wherein a pivot pin (27) pivotably couples the first jaw member to the actuating member, and
wherein the clamp housing includes a recessed track (28) that extends generally parallel to the closing direction, and the pivot pin extends beyond the first jaw member into the clamp housing's recessed track, the pivot pin being configured to slide within the clamp housing's recessed track to permit the actuating member and the first jaw member to translate relative to the clamp housing.

13. A method of attaching an accessory to a bedrail of a surgical/medical bed at a desired location without having to slide the accessory from an end of the bedrail to the desired location, the method comprising:
(a) providing the bedrail clamp of any of the preceding claims;
(b) ensuring that the actuating member is in the clamp-open position and the first jaw member is in a first jaw-open position;
(c) introducing the bedrail into the clamp housing's throat area without obstruction from the first jaw member;
(d) moving the actuating member in a closing direction from the clamp-open position to the clamp-closed position, thereby repositioning the first jaw member into the first-jaw-closed position.

14. The method of claim 13, wherein (i) the bedrail clamp further includes a second jaw member coupled to the actuating member and housed by the clamp housing, the second jaw member comprising third and fourth bedrail portions, (ii) introducing the bedrail into the clamp housing's throat area is without obstruction from the second jaw member, (iii) moving the actuating member in the closing direction from the clamp-open position to the clamp closed position repositions the second jaw member into a second-jaw-closed position in which the clamp housing's first bedrail surface engages the generally vertical outer surface of the bedrail, the second jaw member's third bedrail portion is positioned proximate to whichever of the generally horizontal upper or lower surfaces of the bedrail is not positioned proximate to the first jaw member's first bedrail portion when the first jaw member is in the first jaw-closed position and the second jaw member's fourth bedrail portion engages the generally vertical inner surface of the bedrail.

15. The method of claim 13, wherein repositioning the first jaw member from the first-jaw-open position to the first-jaw-closed position comprises the intermediate step of repositioning the first jaw member from the first-jaw-open position to the first-jaw-partially-closed position, and wherein repositioning the first jaw member from the first-jaw-partially-closed position to the first-jaw-closed position comprises only translating the first jaw member; and/or
repositioning the second jaw member from the second-jaw-open position to the second-jaw-closed position comprises the intermediate step of repositioning the second jaw member from the second-jaw-open position to the second-jaw-partially-closed position, and wherein repositioning the second jaw member from the second-jaw-partially-closed position to the second-jaw-closed position comprises only translating the second jaw member.

## Patentansprüche

1. Bettschienenkleinme (5) zum Befestigen eines Zubehörteils an einer Bettschiene (10) eines medizinischen Betts beziehungsweise eines Operationsbetts in einer gewünschten Position ohne die Bettschienenklemme von einem Ende der Bettschiene schieben zu müssen, bis das Zubehörteil in der gewünschten Position ist, wobei die Bettschienenklemme umfasst:
(a) ein Klemmgehäuse (15), das (i) einen Halsbereich (30) mit einer ersten Bettschienenoberfläche (35) beinhaltet, der angepasst ist, die Bettschiene aufzunehmen, sowie (ii) ein erstes Gehäuseführungselement (40);
(b) ein Betätigungselement (20), das an dem Klemmgehäuse gekoppelt vorliegt und angepasst ist, sich relativ zu dem Klemmgehäuse zwischen einer Position zu bewegen, in der die Klemme geöffnet ist und einer Position, in der die Klemme geschlossen ist, wobei, wenn sich die Bettschiene in dem Halsbereich des Klemmgehäuses befindet, das Betätigungselement in der Klemme geöffnet Position näher an der Bettschiene liegt als in der Klemme geschlossen Position;
(c) eine erste Klemmbacke (25), die mit dem Betätigungselement gekoppelt vorliegt und von dem Klemmgehäuse beherbergt wird, wobei die erste Klemmbacke (i) erste (45) und zweite (50) Bettschienenbereiche und (ii) ein erstes Klemmbackenführungselement (55) beinhaltet, das angepasst ist, körperlich mit dem ersten Gehäuseführungselement des Klemmgehäuses gekoppelt zu werden, wenn sich das Betätigungselement in einer Schließrichtung von der Klemme geöffnet Position zu der Klemme geschlossen Position bewegt, um die Position der ersten Klemmbacke von (A) einer erste Backe geöffnet Position, in der die erste Klemmbacke die Bettschiene nicht daran hindert, in den Halsbereich des Klemmgehäuses hineinzugelangen oder ihn zu verlassen, zu (B) einer erste Backe teilweise geschlossen Position und dann, über lediglich ein Umsetzen der ersten Klemmbacke in die Schließrichtung, zu (C) einer erste Backe geschlossen Position zu verändern, in der, wenn sich die Bettschiene in dem Halsbereich des Klemmgehäuses befindet, die erste Bettschienenoberfläche des Klemmgehäuses in eine allgemein vertikale äußere Oberfläche (60) der Bettschiene eingreift, der erste Bettschienenbereich (45) der ersten Klemmbacke dicht an einer allgemein horizontalen oberen (65) oder unteren (70) Oberfläche der Bettschiene angeordnet ist, und der zweite Bettschienenbereich (50) der Klemmbacke in eine allgemein vertikale innere Oberfläche (75) der Bettschiene eingreift.

2. Bettschienenklemme nach Anspruch 1, wobei (i) der Halsbereich des Klemmgehäuses ferner eine zweite Bettschienenoberfläche (36) umfasst, und (ii) wenn sich die erste Klemmbacke in der erste Backe geschlossen Position befindet und wenn sich die Bettschiene in dem Halsbereich des Klemmgehäuses befindet, die zweite Bettschienenoberfläche des Klemmgehäuses dicht an der allgemein horizontalen oberen oder unteren Oberfläche der Bettschiene angeordnet ist, je nachdem, welche von beiden nicht dicht an dem ersten Bettschienenbereich der ersten Klemmbacke angeordnet ist.

3. Bettschienenklemme nach Anspruch 1, wobei das erste Gehäuseführungselement eine erste vertiefte Schiene (40) umfasst und wobei das erste Klemmbackenführungselement einen ersten Führungsstift (55) umfasst, der angepasst ist, innerhalb der ersten vertieften Schiene zu gleiten, wenn sich das Betätigungselement zwischen der Klemme geöffnet und der Klemme geschlossen Position bewegt.

4. Bettschienenklemme nach Anspruch 3, wobei die erste vertiefte Schiene ein erstes Segment (41) umfasst, das allgemein parallel zu der Schließrichtung liegt, sowie ein zweites Segment (42), das mit dem ersten Segment einen allgemein stumpfen Winkel bildet.

5. Bettschienenklemme nach Anspruch 1, wobei die erste Klemm teilweise geschlossen Position derart ist, dass die erste Klemmbacke die Bettschiene nicht daran hindert, wenn sie sich in dem Halsbereich des Klemmgehäuses befindet, den Halsbereich des Klemmgehäuses zu verlassen, wobei allerdings die erste Bettschienenoberfläche des Klemmgehäuses die allgemein vertikale äußere Oberfläche der Bettschiene und/oder wobei der zweite Bettschienenbereich der ersten Klemmbacke nicht in die allgemein vertikale innere Oberfläche der Bettschiene eingreift.

6. Bettschienenklemme nach Anspruch 1, wobei das Klemmgehäuse ferner umfasst (iii) ein zweites Gehäuse Führungselement, und wobei die Bettschienenklemme ferner umfasst (d) eine zweite Klemmbacke (26), die mit dem Betätigungselement gekoppelt vorliegt und von dem Klemmgehäuse beherbergt wird, wobei die zweite Klemmbacke (i) dritte (46) und vierte (51) Bettschienenbereiche und (ii) ein zweites Klemmbackenführungselement beinhaltet, das angepasst ist, körperlich mit dem zweiten Gehäuseführungselement des Klemmgehäuses gekoppelt zu werden, wenn sich das Betätigungselement in die Schließrichtung von der Klemme geöffnet Position zu der Klemme geschlossen Position bewegt, um die Position der zweiten Klemmbacke über sowohl ein Umsetzen der zweiten Klemmbacke in die Schließrichtung als auch ein Bewegen der zweiten Klemmbacke von (A) einer zweite Backe geöffnet Position, in der die zweite Klemmbacke die Bettschiene nicht daran hindert, in den Halsbereich des Klemmgehäuse hineinzugelangen oder ihn zu verlassen, zu (B) einer zweite Backe geschlossen Position zu verändern, in der, wenn sich die Bettschiene in dem Halsbereich des Klemmgehäuses befindet, die erste Bettschienenoberfläche des Klemmgehäuses in die allgemein vertikale äußere Oberfläche der Bettschiene eingreift, der dritte Bettschienenbereich der zweiten Klemmbacke dicht an derjenigen der allgemein horizontalen oberen oder unteren Oberfläche der Bettschiene angeordnet ist, die nicht dicht an dem ersten Bettschienenbereich der ersten Klemmbacke angeordnet ist, wenn sich die erste Klemmbacke in der ersten Backe geschlossen Position befindet und der vierte Bettschienenbereich der zweiten Klemmbacke in die allgemein vertikale innere Oberfläche der Bettschiene eingreift.

7. Bettschienenklemme nach Anspruch 1, wobei die Schließrichtung allgemein horizontal verläuft.

8. Bettschienenklemme nach Anspruch 1, wobei der erste Bettschienenbereich der ersten Klemmbacke eine erste Oberfläche umfasst, und der zweite Bettschienenbereich der ersten Klemmbacke eine zweite Oberfläche umfasst, die allgemein rechtwinkelig zu der ersten Oberfläche verläuft.

9. Bettschienenklemme nach Anspruch 1, ferner eine Griffanordnung (80) umfassend, die einen Griff (82) und einen Griffstab (84) beinhaltet, der in dem Klemmgehäuse aufgenommen vorliegt, wobei der Griffstab ein proximales Ende (86) aufweist, das mit dem Griff verbunden ist, sowie ein distales Ende (88), das mit dem Betätigungselement (20) verbunden ist.

10. Bettschienenklemme nach Anspruch 9, wobei der Griff einen Knauf umfasst und das distale Ende des Griffstabs über ein Gewinde mit dem Betätigungselement gekoppelt ist, so dass ein Drehen des Knaufs dazu führt, dass sich das Betätigungselement zwischen der Klemme geöffnet Position und der Klemme geschlossen Position bewegt.

11. Bettschienenklemme nach Anspruch 1, wobei die erste Klemmbacke drehbar mit dem Betätigungselement gekoppelt vorliegt, und wobei das erste Klemmbackenführungselement angepasst ist, körperlich mit dem ersten Gehäuseführungselement gekoppelt zu werden, wenn sich das Betätigungselement in der Schließrichtung von der Klemme geöffnet Position zu der Klemme geschlossen Position bewegt, um die Position der ersten Klemmbacke über sowohl ein Umsetzen der ersten Klemmbacke in die Schließrichtung als auch ein Drehen der ersten Klemmbacke von der erste Backe geöffnet Position zu der erste Backe geschlossen Position zu verändern.

12. Bettschienenklemme nach Anspruch 11,
wobei ein Drehstift (27) die erste Klemmbacke an das Betätigungselement koppelt, und wobei das Klemmgehäuse eine vertiefte Schiene (28) beinhaltet, die sich allgemein parallel zur Schließrichtung erstreckt, und wobei sich der Drehstift über die erste Klemmbacke hinaus in die vertiefte Schiene des Klemmgehäuses erstreckt, wobei der Drehstift angepasst ist, in der vertieften Schiene des Klemmgehäuses zu gleiten, um es dem Betätigungselement und der ersten Klemmbacke zu ermöglichen, relativ zu dem Klemmgehäuse versetzt zu werden.

13. Verfahren zum Anordnen eines Zubehörteils an einer Bettschiene eines medizinischen Betts beziehungsweise eines Operationsbetts in einer gewünschten Position ohne das Zubehörteil von einem Ende der Bettschiene zur gewünschten Position schieben zu müssen, wobei das Verfahren umfasst:
(a) Bereitstellen einer Bettschienenklemme nach einem der vorstehenden Ansprüche;
(b) Sicherstellen, dass sich das Betätigungselement in der Klemme geöffnet Position befindet und dass sich die erste Klemmbacke in der ersten Backe geöffnet Position befindet;
(c) Einführen der Bettschiene in den Halsbereich des Klemmgehäuses ohne Behinderung durch die erste Klemmbacke;
(d) Bewegen des Betätigungselements in einer Schließrichtung von der Klemme geöffnet Position zu der Klemme geschlossen Position, dadurch Verändern der Position der ersten Klemmbacke in die erste Backe geschlossen Position.

14. Verfahren nach Anspruch 13, wobei (i) die Bettschienenklemme ferner eine zweite Klemmbacke beinhaltet, die mit dem Betätigungselement gekoppelt vorliegt und von dem Klemmgehäuse beherbergt wird, wobei die zweite Klemmbacke dritte und vierte Bettschienenbereiche umfasst, (ii) Einführen der Bettschiene in den Halsbereich des Klemmgehäuses ohne Behinderung durch die zweite Klemmbacke, (iii) das Bewegen des Betätigungselements in der Schließrichtung von der Klemme geöffnet Position zu der Klemme geschlossen Position verändert die Position der zweiten Klemmbacke in eine zweite Backe geschlossen Position, in der die erste Bettschienenoberfläche des Klemmgehäuse in die allgemein vertikale äußere Oberfläche der Bettschiene eingreift, wobei der dritte Bettschienenbereich der zweiten Klemmbacke dicht an derjenigen der allgemein horizontalen oberen oder unteren Oberfläche der Bettschiene angeordnet ist, die nicht dicht an dem ersten Bettschienenbereich der ersten Klemmbacke angeordnet ist, wenn sich die erste Klemmbacke in der ersten Backe geschlossen Position befindet und der vierte Bettschienenbereich der zweiten Klemmbacke in die allgemein vertikale innere Oberfläche der Bettschiene eingreift.

15. Verfahren nach Anspruch 13, wobei das Verändern der Position der ersten Klemmbacke von der erste Backe offen Position zu der erste Backe geschlossen Position den Zwischenschritt des Veränderns der Position der ersten Klemmbacke von der erste Backe geöffnet Position zu der erste Backe teilweise geschlossen Position beinhaltet, und wobei das Verändern der Position der ersten Klemmbacke von der erste Backe teilweise geschlossen Position zu der erste Backe geschlossen Position lediglich ein Umsetzen der ersten Klemmbacke umfasst; und/oder
wobei ein Verändern der Position der zweiten Klemmbacke von der zweite Backe geöffnet Position zu der zweite Backe geschlossen Position den Zwischenschritt des Veränderns der Position der zweiten Klemmbacke von der zweite Backe geöffnet Position zu der zweite Backe teilweise geschlossen Position beinhaltet, und wobei das Verändern der Position der zweiten Klemmbacke von der zweite Backe teilweise geschlossen Position zu der zweite Backe geschlossen Position lediglich ein Umsetzen der zweiten Klemmbacke umfasst.

## Revendications

1. Attache (5) pour barrière de lit pour fixer un accessoire à une barrière de lit (10) d'un lit chirurgical/médical à un emplacement souhaité sans devoir faire coulisser l'attache de barrière de lit à partir d'une extrémité de la barrière de lit jusqu'à ce que l'accessoire soit à l'emplacement souhaité, l'attache pour barrière de lit comprenant :
(a) un logement d'attache (15) qui comprend (i) une zone de gorge (30) ayant une première surface de barrière de lit (35) et étant configurée pour recevoir la barrière de lit et (ii) un premier élément de guidage de logement (40) ;
(b) un élément d'actionnement (20) couplé au logement d'attache et configuré pour se déplacer par rapport au logement d'attache entre une position ouverte d'attache et une position fermée d'attache, dans laquelle la barrière de lit est dans la zone de gorge du logement d'attache, l'élément d'actionnement est plus près de la barrière de lit dans la position ouverte d'attache que dans la position fermée d'attache ;
(c) un premier élément de mâchoire (25) couplé à l'élément d'actionnement et logé par le logement d'attache, le premier élément de mâchoire comprenant (i) des première (45) et seconde (50) parties de barrière de lit et (ii) un premier élément de guidage d'élément de mâchoire (55) configuré pour s'interfacer physiquement avec le premier élément de guidage de logement du logement d'attache lorsque l'élément d'actionnement se déplace dans une direction de fermeture de la position ouverte d'attache à la position fermée d'attache afin de repositionner le premier élément de mâchoire (A) d'une position ouverte de première mâchoire dans laquelle le premier élément de mâchoire n'empêche pas la barrière de lit d'entrer ou de sortir de la zone de gorge du logement d'attache à (B) une position fermée partiellement de première mâchoire et ensuite, uniquement par la translation du premier élément de mâchoire dans la direction de fermeture, à (C) une position fermée de première mâchoire dans laquelle, lorsque la barrière de lit est dans la zone de gorge du logement d'attache, la première surface de barrière de lit du logement d'attache met en prise une surface externe (60) généralement verticale de la barrière de lit, la première partie de barrière de lit (45) du premier élément de mâchoire est positionnée à proximité d'une surface supérieure (65) ou inférieure (70) généralement horizontale de la barrière de lit, et la seconde partie de barrière de lit (50) du premier élément de mâchoire met en prise une surface interne (75) généralement verticale de la barrière de lit.

2. Attache pour barrière de lit selon la revendication 1, dans laquelle (i) la zone de gorge du logement d'attache a en outre une seconde surface de barrière de lit (36) et (ii) lorsque le premier élément de mâchoire est dans la position fermée de première mâchoire et lorsque la barrière de lit est dans la zone de gorge du logement d'attache, la seconde surface de barrière de lit du logement d'attache est positionnée à proximité de l'une quelconque des surfaces supérieures ou inférieures généralement horizontales de la barrière de lit qui n'est pas positionnée à proximité de la première partie de barrière de lit du premier élément de mâchoire.

3. Attache pour barrière de lit selon la revendication 1, dans laquelle le premier élément de guidage de logement comprend un premier rail évidé (40) et le premier élément de guidage d'élément de mâchoire comprend une première broche de guidage (55) configurée pour coulisser à l'intérieur du premier rail évidé, lorsque l'élément d'actionnement se déplace entre le position ouverte d'attache et la position fermée d'attache.

4. Attache pour barrière de lit selon la revendication 3, dans laquelle le premier rail évidé comprend un premier segment (41) qui est généralement parallèle à la direction de fermeture et un second segment (42) qui forme un angle généralement obtus avec le premier segment.

5. Attache pour barrière de lit selon la revendication 1, dans laquelle la position partiellement fermée de première mâchoire est telle que le premier élément de mâchoire n'empêche pas la barrière de lit, lorsqu'elle est positionnée dans la zone de gorge du logement d'attache, de sortir de la zone de gorge de logement d'attache, mais la première surface de barrière de lit du logement d'attache ne met pas en prise la surface externe généralement verticale de la barrière de lit et/ou la deuxième partie de barrière de lit du premier élément de mâchoire ne met pas en prise la surface interne généralement verticale de la barrière de lit.

6. Attache pour barrière de lit selon la revendication 1, dans laquelle le logement d'attache comprend en outre (iii) un second élément de guidage de logement, et dans laquelle l'attache de barrière de lit comprend en outre (d) un second élément de mâchoire (26) couplé à l'élément d'actionnement et logé par le logement d'attache, le second élément de mâchoire comprenant (i) des troisième (46) et quatrième (51) parties de barrière de lit et (ii) un second élément de guidage d'élément de mâchoire configuré pour s'interfacer physiquement avec le second élément de guidage de logement du logement d'attache lorsque l'élément d'actionnement passe dans la direction de fermeture, de la position ouverte d'attache à la position fermée d'attache afin de repositionner le second élément de mâchoire, à la fois en faisant effectuer un mouvement de translation au second élément de mâchoire dans la direction de fermeture et en déplaçant le second élément de mâchoire, depuis (A) une position ouverte de seconde mâchoire dans laquelle le second élément de mâchoire n'empêche pas la barrière de lit d'entrer ou de sortir de la zone de gorge du logement d'attache à (B) une position fermée de seconde mâchoire dans laquelle, lorsque la barrière de lit est dans la zone de gorge du logement d'attache, la première surface de barrière de lit du logement d'attache met en prise la surface externe généralement verticale de la barrière de lit, la troisième partie de barrière de lit du second élément de mâchoire est positionnée à proximité de l'une quelconque des surfaces supérieures ou inférieures généralement horizontales de la barrière de lit qui n'est pas positionnée à proximité de la première partie de barrière de lit du premier élément de mâchoire lorsque le premier élément de mâchoire est dans la position fermée de première mâchoire, et la quatrième partie de barrière de lit du second élément de mâchoire met en prise la surface interne généralement verticale de la barrière de lit.

7. Attache pour barrière de lit selon la revendication 1, dans laquelle la direction de fermeture est généralement horizontale.

8. Attache pour barrière de lit selon la revendication 1, dans laquelle la première partie de barrière de lit du premier élément de mâchoire comprend une première surface et la deuxième partie de barrière de lit du premier élément de mâchoire comprend une seconde surface qui est généralement perpendiculaire à la première surface.

9. Attache pour barrière de lit selon la revendication 1, comprenant en outre un ensemble de poignée (80) qui comprend une poignée (82) et un arbre de poignée (84) logé par le logement d'attache, l'arbre de poignée ayant une extrémité proximale (86) couplée à la poignée et une extrémité distale (88) couplée à l'élément d'actionnement (20).

10. Attache pour barrière de lit selon la revendication 9, dans laquelle la poignée comprend un bouton et l'extrémité distale de l'arbre de poignée est couplée par filetage à l'élément d'actionnement de sorte que le pivotement du bouton provoque le déplacement de l'élément d'actionnement entre la position ouverte d'attache et la position fermée d'attache.

11. Attache pour barrière de lit selon la revendication 1, dans laquelle le premier élément de mâchoire est couplé, de manière pivotante, à l'élément d'actionnement et le premier élément de guidage d'élément de mâchoire est configuré pour s'interfacer physiquement avec le premier élément de guidage de logement lorsque l'élément d'actionnement passe dans la direction de fermeture de la position ouverte d'attache à la position fermée d'attache afin de repositionner le premier élément de mâchoire, à la fois en faisant effectuer un mouvement de translation au premier élément de mâchoire dans la direction de fermeture et en faisant pivoter le premier élément de mâchoire, de la position ouverte de première mâchoire à la position fermée de première mâchoire.

12. Attache pour barrière de lit selon la revendication 11, dans laquelle une broche de pivot (27) couple, de manière pivotante, le premier élément de mâchoire à l'élément d'actionnement, et
dans laquelle le logement d'attache comprend un rail évidé (28) qui s'étend généralement parallèlement à la direction de fermeture, et la broche de pivot s'étend au-delà du premier élément de mâchoire dans le rail évidé du logement d'attache, la broche de pivot étant configurée pour coulisser à l'intérieur du rail évidé du logement d'attache pour permettre à l'élément d'actionnement et au premier élément de mâchoire d'effectuer un mouvement de translation par rapport au logement d'attache.

13. Procédé pour fixer un accessoire à une barrière de lit d'un lit chirurgical / médical à un emplacement souhaité sans devoir faire coulisser l'accessoire d'une extrémité de la barrière de lit à l'emplacement souhaité, le procédé comprenant les étapes consistant à :
(a) prévoir l'attache de barrière de lit selon l'une quelconque des revendications précédentes ;
(b) s'assurer que l'élément d'actionnement est dans la position ouverte d'attache et que le premier élément de mâchoire est dans une position ouverte de première mâchoire ;
(c) introduire la barrière de lit dans la zone de gorge du logement d'attache sans obstruction du premier élément de mâchoire ;
(d) faire passer l'élément d'actionnement dans une direction de fermeture de la position ouverte d'attache à la position fermée d'attache, repositionnant ainsi le premier élément de mâchoire dans la position fermée de première mâchoire.

14. Procédé selon la revendication 13, dans lequel (i) l'attache de barrière de lit comprend un second élément de mâchoire couplé à l'élément d'actionnement et logé par le logement d'attache, le second élément de mâchoire comprenant des troisième et quatrième parties de barrière de lit, (ii) l'introduction de la barrière de lit dans la zone de gorge du logement d'attache se fait sans obstruction du second élément de mâchoire, (iii) le déplacement de l'élément d'actionnement dans la direction de fermeture de la position ouverte d'attache à la position fermée d'attache repositionne le second élément de mâchoire dans une position fermée de seconde mâchoire dans laquelle la première surface de barrière de lit du logement d'attache met en prise la surface externe généralement verticale de la barrière de lit, la troisième partie de barrière de lit du second élément de mâchoire est positionnée à proximité de l'une quelconque des surfaces supérieures ou inférieures généralement horizontales de la barrière de lit qui n'est pas positionnée à proximité de la première partie de barrière de lit du premier élément de mâchoire lorsque le premier élément de mâchoire est dans la position fermée de première mâchoire et la quatrième partie de barrière de lit du second élément de mâchoire met en prise la surface interne généralement verticale de la barrière de lit.

15. Procédé selon la revendication 13, dans lequel l'étape consistant à repositionner le premier élément de mâchoire de la position ouverte de première mâchoire à la position fermée de première mâchoire comprend l'étape intermédiaire consistant à repositionner le premier élément de mâchoire de la position ouverte de première mâchoire à la position partiellement fermée de première mâchoire et dans lequel l'étape consistant à repositionner le premier élément de mâchoire de la position partiellement fermée de première mâchoire à la position fermée de première mâchoire comprend l'étape consistant à faire effectuer un mouvement de translation uniquement au premier élément de mâchoire ; et/ou
l'étape consistant à repositionner le second élément de mâchoire de la position ouverte de seconde mâchoire à la position fermée de seconde mâchoire comprend l'étape intermédiaire consistant à repositionner le second élément de mâchoire de la position ouverte de seconde mâchoire à la position partiellement fermée de seconde mâchoire, et dans lequel l'étape consistant à repositionner le second élément de mâchoire de la position partiellement fermée de seconde mâchoire à la position fermée de seconde mâchoire comprend l'étape consistant à faire effectuer un mouvement de translation uniquement au second élément de mâchoire.
